# EUROPEAN PATENT SPECIFICATION

(11) **EP 3 884 841 B1**
(45) Date of publication and mention of the grant of the patent: **29.05.2024**
(21) Application number: 21163829.1
(22) Date of filing: 19.03.2021
(51) Int. Cl.: A61B 1/015, A61M 1/00, A61B 1/303, A61B 46/23

(54) **OUTFLOW COLLECTION SYSTEMS FOR HYSTEROSCOPIC SURGICAL PROCEDURES**
ABFLUSSSAMMELSYSTEME FÜR HYSTEROSKOPISCHE CHIRURGISCHE VERFAHREN
SYSTÈMES DE COLLECTE DE SORTIE POUR DES PROCÉDURES CHIRURGICALES HYSTÉROSCOPIQUES

(30) Priority: 25.03.2020 US 202062994401 P; 23.12.2020 US 202017132746
(43) Date of publication of application: 29.09.2021
(73) Proprietor: Covidien LP, Mansfield, MA 02048 (US)
(72) Inventor: CROSS, Damian J., Woburn, MA 01801 (US); WHISLER, Jordan A., Woburn, MA 01801 (US); MARINKOVIC, Aleksandar, Woburn, MA 01801 (US); RYBICKI, Steven M., Woburn, MA 01801 (US)
(74) Representative: Zimmermann & Partner Patentanwälte mbB

(56) References cited:
- WO-A1-2012/087376
- US-A- 5 928 249
- US-A1- 2012 172 888
- US-A1- 2017 000 946
- US-A1- 2020 069 851
- US-B1- 6 938 639

## Description

### BACKGROUND

### Technical Field

The present disclosure relates generally to surgical systems and, more particularly, to outflow collection systems for hysteroscopic surgical procedures present disclosure relates generally to surgical systems and, more particularly, to outflow collection systems for hysteroscopic surgical procedures.

### Background of Related Art

Surgical procedures, such as hysteroscopic surgical procedures, may be performed endoscopically within an organ, such as a uterus, by inserting an endoscope into the uterus and passing a tissue resection device through the endoscope and into the uterus. With respect to such hysteroscopic tissue resection procedures, it often is desirable to distend the uterus with a liquid, for example, saline, sorbitol, or glycine. The inflow and outflow of the liquid during the procedure maintains the uterus in a distended state and flushes tissue and other debris from within the uterus to maintain a visible working space. The outflow liquid is collected by a collection system,

Document US 2012/172888 A1 relates to a hysteroscopic tissue removal system with improved fluid management and/or monitoring capabilities.

Document US 6,938,639 B1 relates to a disposable fluid control island.

Document US 2017/0000946 A1 relates to a fluid infusion system.

### SUMMARY

The invention is defined in the appended claims. Aspects, embodiments and examples disclosed herein which do not fall within the scope of the appended claims do not form part of the invention, and are merely provided for illustrative purposes.

As used herein, the term "distal" refers to the portion that is described which is farther from a user, while the term "proximal" refers to the portion that is described which is closer to a user. Further, to the extent consistent, any or all of the aspects described herein may be used in conjunction with any or all of the other aspects described herein.

Provided in accordance with aspects of the present disclosure is a collection system for collecting outflow from a hysteroscopic surgical procedure. The collection system includes a surgical drape configured to collect liquid, a collection vessel, a vacuum source, a drape line connected between the surgical drape and the collection vessel, a vacuum line connected between the vacuum source and the collection vessel, and a filter assembly. A liquid flow path is defined from the surgical drape, through the drape line, and to the collection vessel. The vacuum source is configured to establish suction to draw liquid from the surgical drape along the liquid flow path. The filter assembly is disposed along the liquid flow path and includes a hydrophilic membrane configured to permit passage of liquid along the liquid flow path and impede passage of air along the along the liquid flow path.

In an aspect of the present disclosure, the filter assembly further includes a hydrophobic membrane. In such aspects, the hydrophilic membrane may be relatively large while the hydrophobic membrane is relatively small.

In another aspect of the present disclosure, the filter assembly further includes a body housing the hydrophilic membrane therein. The body defines an input and an output disposed along the liquid flow path.

In yet another aspect of the present disclosure, the filter assembly is disposed between the drape line and the surgical drape. Alternatively, the filter assembly is disposed between the drape line and the collection vessel. Further still, the filter assembly may be disposed within the drape line, e.g., towards a drape line end thereof or towards a collection vessel end thereof.

In still another aspect of the present disclosure, a flow restrictor is disposed along the liquid flow path in parallel with the filter assembly.

In aspects, the drape line includes an upstream portion connecting the surgical drape with the input of the chamber and a downstream portion connecting the output of the chamber with the collection vessel.

### BRIEF DESCRIPTION OF THE DRAWINGS

Various aspects and features of the present disclosure are described hereinbelow with reference to the drawings wherein like numerals designate identical or corresponding elements in each of the several views.
FIG. I is a schematic illustration of an outflow collection system in use during a hysteroscopic surgical procedure in accordance with the present disclosure;
FIG. 2 is a side view of a portion of a drape and drape line of the collection system of FIG. 1 including a filter in accordance with the present disclosure,
FIG. 3A is a side view of the filter of FIG. 2;
FIG. 3B is an exploded, perspective view of the filter of FIG. 2;
FIG. 4A is a perspective view of a collection vessel of the outflow collection system of FIG. 1 including another filter in accordance with the present disclosure;
FIG. 4B is an exploded, perspective view of the filter of FIG. 4A; and
FIG. 5 is a schematic illustration of another outflow collection system in use during a hysteroscopic surgical procedure in accordance with the present disclosure.

### DETAILED DESCRIPTION

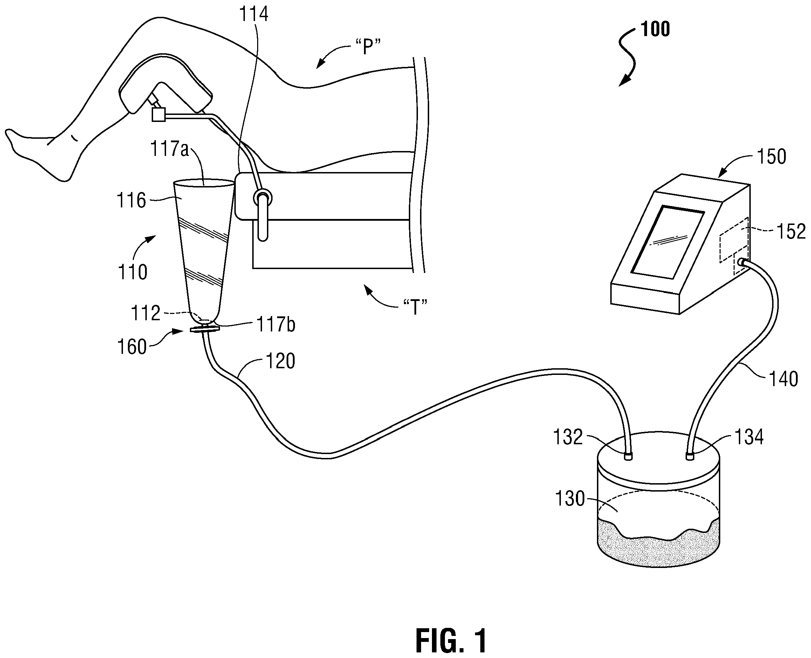

Referring to FIG. 1, an outflow collection system provided in accordance with aspects of the present disclosure is shown generally identified by reference numeral 100. Outflow collection system 100 includes a surgical drape 110, a drape line 120, a collection vessel 130, a vacuum line 140, and a vacuum source, e.g., a control console 150 including a vacuum pump 152. Outflow collection system 100 further includes a filter assembly 160 disposed between the outflow opening 112 of surgical drape 110 and drape line 120 or disposed within drape line 120 towards the drape-end thereof (e.g., within 10% of a length of drape line 120 from the drape-end of drape line 120). Other suitable positions for filter assembly 160 are also contemplated. Outflow collection system 100 may further include one or more instrument lines (not shown) connected between one or more surgical instruments (not shown), e.g., an hysteroscope, a tissue resection device, etc., and the collection vessel 130 or an additional collection vessel.

Continuing with reference to FIG. 1, surgical drape 110 is positioned with a flap 114 thereof positioned underneath a patient "P," e.g., between the patient "P" and the operating table "T," with a funnel-shaped body 116 of the surgical drape 110 depending from the flap 114 and an end of the operating table "T." The funnel-shaped body 116 defines an open, upper base end 117a configured to collect liquid, e.g., dripping out of the patient "P" and/or onto the operating table "T," and a lower, apex end 117b that defines outflow opening 112, through which the collected liquid is configured to flow. An input 164 of filter assembly 160 is connected to outflow opening 112 while the drape-end of drape line 120 is connected to an output 166 of filter assembly 160. Alternatively, the drape-end of drape line 120 may be connected to outflow opening 112 with filter assembly 160 disposed along drape line 120 towards the drape-end thereof, as noted above.

Drape line 120 extends to collection vessel 130 and is connected to a first port 132 of collection vessel 130. Collection vessel 130 further includes a second port 134 configured to receive an end of vacuum line 140. The other end of vacuum line 140 is coupled to a vacuum source, e.g., vacuum pump 152 of control console 150, such that, upon activation of vacuum pump 152, negative pressure is established through collection vessel 130 and drape line 120 to draw liquids collected in surgical drape 110 from surgical drape 110, through drape line 120, to collection vessel 130. However, if there are no liquids or minimal liquids in surgical drape 110, outflow opening 112 of surgical drape 110 may become open to the atmosphere, e.g., via the open, upper base end 117a of surgical drape 110, which could result in the suctioning of air through surgical drape 110, drape line 120, and into collection vessel 130. The result is loss of suction through other channels, e.g., through the one or more instrument lines (not shown) connected between the one or more surgical instruments (not shown) and the collection vessel 130, as the vacuum pump 152 becomes dedicated to the path of least resistance, which results in all suction being applied to suction the air into collection vessel 130.

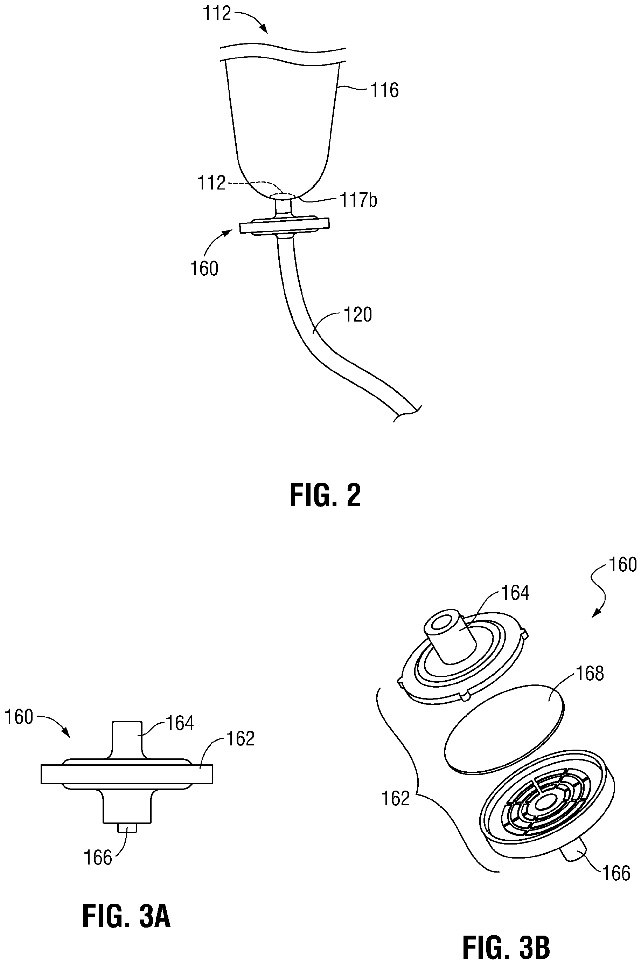

With additional reference to FIGS. 2-3B, in order to inhibit the suctioning of air through surgical drape 110, drape line 120, and into collection vessel 130 and, thus, loss of suction through other channels, filter assembly 160 is provided between the outflow opening 112 of surgical drape 110 and drape line 120 or disposed within drape line 120 towards the drape-end thereof. Filter assembly 160 includes a body 162 including input 164 and output 166 and housing a membrane 168 therebetween. Membrane 168 is a hydrophilic membrane that permits liquid, e.g., water and water-based liquid, to flow therethrough but inhibits (to a threshold) or impedes (beyond the threshold) the flow of air therethrough. In this manner, the drawing, under suction, of liquids collected in surgical drape 110 from surgical drape 110, through drape line 120, to collection vessel 130 is maintained, while sufficient resistance is provided to inhibit or limit the suctioning of air through membrane 168 in order to maintain suction through the other channels when there are no liquids or minimal liquids in surgical drape 110.

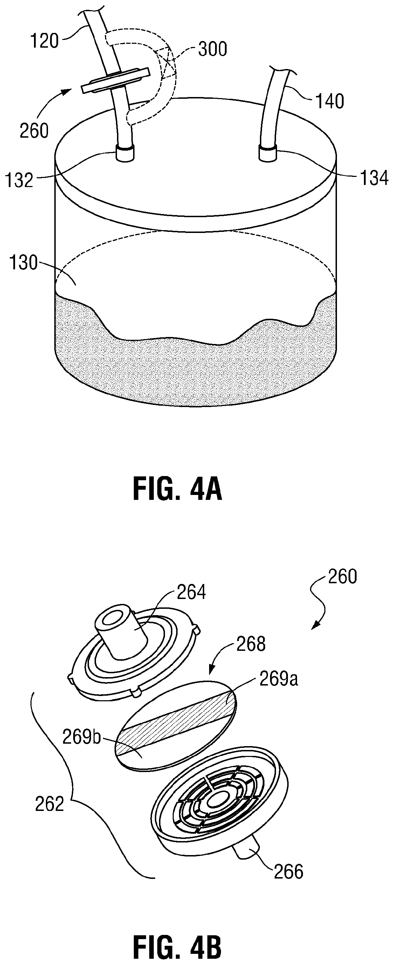

Turning to FIGS. 4A and 4B, in other embodiments, a filter assembly 260 may be disposed between drape line 120 and first port 132 of collection vessel 130 or disposed within drape line 120 towards the collection vessel-end thereof (e.g., within 10% of a length of drape line 120 from the collection vessel-end of drape line 120). Other suitable positions for filter assembly 160 are also contemplated. Filter assembly 260 may include a body 262 including an input 264 and an output 266 and housing a membrane 268 therebetween. In embodiments, membrane 268 may be a hydrophilic membrane similarly as detailed above with respect to filter assembly 160 (FIG. 3B), or may be a combined hydrophilic/hydrophobic membrane. Further, in additional or alternative embodiments, a flow restrictor 300 may be disposed in parallel with membrane 268 to purge air while permitting liquid flow through membrane 268 into collection vessel 230.

In embodiments where membrane 268 is a combined hydrophilic/hydrophobic membrane and/or where flow restrictor 300 is utilized, the need for maintaining the positioning of filter assembly 260 below the liquid level within surgical drape 110 (FIG. 1) is obviated. However, it is also contemplated that membrane 268, configured as a hydrophilic/hydrophobic membrane, and/or flow restrictor 300 be utilized at other locations, including those where filter assembly 260 is maintained below the liquid level within surgical drape 110 (FIG. 1), e.g., at or towards outflow opening 112 of surgical drape 110.

Referring to FIG. 4B, with respect to embodiments where membrane 268 is a combined hydrophilic/hydrophobic membrane, membrane 268 may be formed by welding a hydrophobic membrane strip 269a onto a hydrophilic membrane body 269b, or in any other suitable manner. In such a configuration, in use, air is purged through the relatively smaller hydrophobic membrane strip 269a but is inhibited or impeded from passing through the relatively larger hydrophilic membrane body 269b. Liquid, on the other hand, passes around the relatively smaller hydrophobic membrane strip 269a and into contact with the relatively larger hydrophilic membrane body 269b, which permits the liquid to flow therethrough and continue out the output of 266 of body 262 of filter assembly 260 into collection vessel 130. The above configuration enables liquid flow while inhibiting or impeding air flow due to the fact that a relatively high flow rate of liquid through the relatively larger hydrophilic membrane body 269b is created as compared to the relatively low flow rate of air through the relatively smaller hydrophobic membrane strip 269a. Thus, liquid is suctioned first before air and the flow rate of air is sufficiently reduced such that, in the event there are no liquids or minimal liquids (remaining) in surgical drape 110 (FIG. 1), suction through the other channels is maintained.

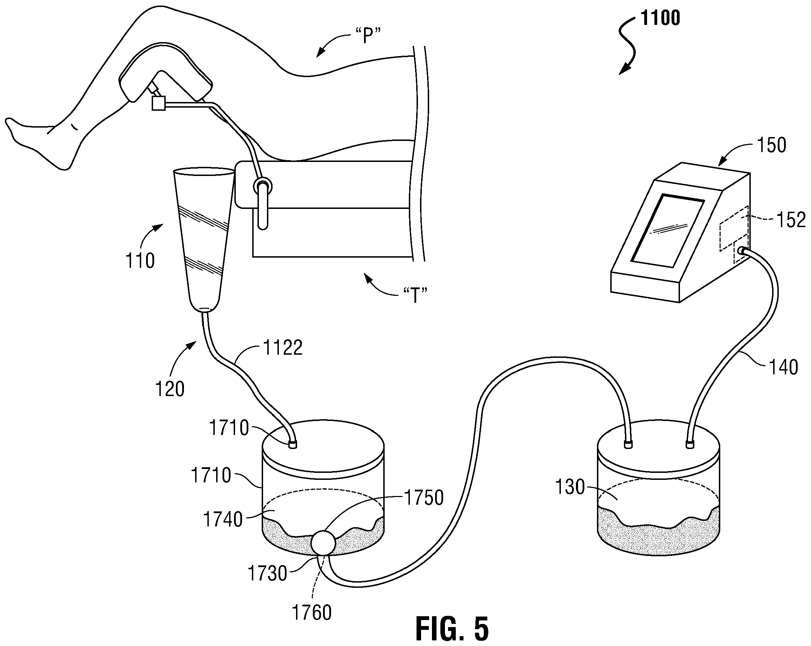

With reference to FIG. 5, another embodiment of an outflow collection system 1100 includes surgical drape 110, drape line 120, collection vessel 130, vacuum line 140, and a vacuum source, e.g., control console 150 including vacuum pump 152. With respect to outflow collection system 1100, drape line 120 includes an upstream drape line portion 1122 and a downstream drape line portion 1224. A valve assembly 1700 is disposed between the upstream drape line portion 1122 and the downstream drape line portion 1224, although valve assembly 1700 may alternatively be disposed before or after drape line 120. Valve assembly 1700 includes a chamber 1710 having an input 1720 and an output 1730 and an interior volume 1740 defined between the input 1720 and the output 1730. A buoyant float ball 1750 is positioned to selectively open and close an output aperture 1760 leading to the output 1730. More specifically, when the level of liquid within interior volume 1740 is sufficiently high, the buoyant float ball 1750 is displaced from the output aperture 1760, permitting liquid to flow from the interior volume 1740, through the output aperture 1760 and through the output 1730 to the downstream drape line portion 1224 and, ultimately, to collection vessel 130. On the other hand, when the level of liquid within interior volume 1740 is not sufficiently high, buoyant float ball 1750 remains in position blocking output aperture 1760 and inhibiting fluid, e.g., liquid or air, from passing through output aperture 1760 to the downstream drape line portion 1224. Thus, only when a sufficient amount of liquid is collected by surgical drape 110 and flows through upstream drape line portion 1122 into interior volume 1740 of chamber 1710 so as to displace buoyant float ball 1750, is liquid permitted to flow through output aperture 1760. When there is an insufficient amount of liquid, buoyant float ball 1750 blocks output aperture 1760. Thus, suctioning is substantially limited to liquid; no substantial suctioning of atmospheric air occurs, even when where is little or no liquid. Thus, suction is maintained through the other channels in the instances where is little or no liquid.

## Claims

1. A collection system (100) for collecting outflow from a hysteroscopic surgical procedure, the collection system (100) comprising:
a surgical drape (110) configured to collect liquid;
a collection vessel (130);
a vacuum source;
**characterized in that** the system further comprises:
a drape line (120) connected between the surgical drape (110) and the collection vessel (130), wherein a liquid flow path is defined from the surgical drape (110), through the drape line (120), and to the collection vessel (130);
a vacuum line (140) connected between the vacuum source and the collection vessel (130), the vacuum source configured to establish suction to draw liquid from the surgical drape (110) along the liquid flow path; and
- a filter assembly (160) disposed along the liquid flow path, the filter assembly (160) including a hydrophilic membrane configured to permit passage of liquid along the liquid flow path and impede passage of air along the along the liquid flow path,
or
- a valve assembly (1700) disposed along the liquid flow path, the valve assembly including a chamber defining an input configured to receive liquid flowing along the liquid flow path and an output configured to output fluid along the liquid flow path, the valve assembly including a buoyant float ball disposed within the chamber and configured to selectively open and close the output based upon a level of liquid the chamber, thereby inhibiting air from flowing out the output.

2. The collection system (100) according to claim 1, wherein, when the collection system (100) comprises the filter assembly (160), the filter assembly (160) further includes a hydrophobic membrane.

3. The collection system (100) according to claim 1 or 2 wherein, when the collection system (100) comprises the filter assembly (160), the filter assembly (160) further includes a body housing the hydrophilic membrane therein, the body defining an input and an output disposed along the liquid flow path.

4. The collection system (100) according to any preceding claim, wherein, when the collection system (100) comprises the filter assembly (160), the filter assembly (160) is disposed between the drape line (120) and the surgical drape (110).

5. The collection system (100) according to any preceding claim, wherein, when the collection system (100) comprises the filter assembly (160), the filter assembly (160) is disposed between the drape line (120) and the collection vessel (130).

6. The collection system (100) according to any preceding claim, wherein, when the collection system (100) comprises the filter assembly (160), the filter assembly (160) is disposed within the drape line (120) towards a drape line end thereof or towards a collection vessel end thereof.

7. The collection system (100) according to any preceding claim, further comprising a flow restrictor disposed along the liquid flow path in parallel with the filter assembly (160), when the collection system (100) comprises the filter assembly (160).

8. The collection system (100) according to claim 1, wherein, when the collection system (100) comprises the valve assembly (1700), the drape line (120) includes an upstream portion connecting the surgical drape (110) with the input of the chamber and a downstream portion connecting the output of the chamber with the collection vessel (130).

## Patentansprüche

1. Sammelsystem (100) zum Sammeln von Ausfluss aus einem hysteroskopischen Operationsverfahren, das Sammelsystem (100) umfassend:
eine chirurgische sterile Einweghülle (110), die konfiguriert ist, um Flüssigkeit zu sammeln;
ein Sammelbehältnis (130);
eine Vakuumquelle;
**dadurch gekennzeichnet, dass** das System ferner umfasst:
eine Leitung (120) der sterilen Einweghülle, die zwischen der chirurgischen sterilen Einweghülle (110) und dem Sammelbehältnis (130) verbunden ist, wobei ein Flüssigkeitsströmungsweg aus der chirurgischen sterilen Einweghülle (110) durch die Leitung (120) der sterilen Einweghülle und zu dem Sammelbehältnis (130) definiert ist;
eine Vakuumleitung (140), die zwischen der Vakuumquelle und dem Sammelbehältnis (130) verbunden ist, wobei die Vakuumquelle konfiguriert ist, um einen Sog herzustellen, um Flüssigkeit aus der chirurgischen sterilen Einweghülle (110) entlang des Flüssigkeitsströmungswegs zu ziehen; und
- eine Filterbaugruppe (160), die entlang des Flüssigkeitsströmungswegs angeordnet ist, wobei die Filterbaugruppe (160) eine hydrophile Membran einschließt, die konfiguriert ist, um einen Durchgang von Flüssigkeit entlang des Flüssigkeitsströmungswegs zu ermöglichen und den Durchgang von Luft entlang des Flüssigkeitsströmungswegs zu behindern,
oder
- eine Ventilbaugruppe (1700), die entlang des Flüssigkeitsströmungswegs angeordnet ist, wobei die Ventilbaugruppe eine Kammer einschließt, die einen Eingang definiert, der konfiguriert ist, um Flüssigkeit aufzunehmen, die entlang des Flüssigkeitsströmungswegs fließt, und einen Ausgang, der konfiguriert ist, um Fluid entlang des Flüssigkeitsströmungswegs auszugeben, wobei die Ventilbaugruppe eine schwimmfähige Schwimmkugel einschließt, die innerhalb der Kammer angeordnet und konfiguriert ist, um den Ausgang selektiv zu öffnen und zu schließen, basierend auf einem Flüssigkeitsfüllstand der Kammer, wodurch verhindert wird, dass Luft aus dem Ausgang strömt.

2. Sammelsystem (100) nach Anspruch 1, wobei, wenn das Sammelsystem (100) die Filterbaugruppe (160) umfasst, die Filterbaugruppe (160) ferner eine hydrophobe Membran einschließt.

3. Sammelsystem (100) nach Anspruch 1 oder 2, wobei, wenn das Sammelsystem (100) die Filterbaugruppe (160) umfasst, die Filterbaugruppe (160) ferner einen Körper einschließt, der die hydrophile Membran darin aufnimmt, wobei der Körper einen Eingang und einen Ausgang definiert, die entlang des Flüssigkeitsströmungswegs angeordnet sind.

4. Sammelsystem (100) nach einem der vorstehenden Ansprüche, wobei, wenn das Sammelsystem (100) die Filterbaugruppe (160) umfasst, die Filterbaugruppe (160) zwischen der Leitung (120) der sterilen Einweghülle und der chirurgischen sterilen Einweghülle (110) angeordnet ist.

5. Sammelsystem (100) nach einem der vorstehenden Ansprüche, wobei, wenn das Sammelsystem (100) die Filterbaugruppe (160) umfasst, die Filterbaugruppe (160) zwischen der Leitung (120) der sterilen Einweghülle und dem Sammelbehältnis (130) angeordnet ist.

6. Sammelsystem (100) nach einem der vorstehenden Ansprüche, wobei, wenn das Sammelsystem (100) die Filterbaugruppe (160) umfasst, die Filterbaugruppe (160) innerhalb der Leitung (120) der sterilen Einweghülle zu einem Ende von der Leitung der sterilen Einweghülle hin oder zu einem Ende von dem Sammelbehältnis hin angeordnet ist.

7. Sammelsystem (100) nach einem der vorstehenden Ansprüche, ferner umfassend einen Durchflussbegrenzer, der entlang des Flüssigkeitsströmungswegs parallel zu der Filterbaugruppe (160) angeordnet ist, wenn das Sammelsystem (100) die Filterbaugruppe (160) umfasst.

8. Sammelsystem (100) nach Anspruch 1, wobei, wenn das Sammelsystem (100) die Ventilbaugruppe (1700) umfasst, die Leitung (120) der sterilen Einweghülle einen stromaufwärtigen Abschnitt, der die chirurgische sterile Einweghülle (110) mit dem Eingang der Kammer verbindet, und einen stromabwärtigen Abschnitt einschließt, der den Ausgang der Kammer mit dem Sammelbehältnis (130) verbindet.

## Revendications

1. Système de collecte (100) pour collecter un écoulement d'une procédure chirurgicale hystéroscopique, le système de collecte (100) comprenant :
un champ opératoire (110) conçu pour collecter un liquide ;
un récipient de collecte (130) ;
une source de vide ;
**caractérisé en ce que** le système comprend en outre :
une ligne de champ (120) connectée entre le champ opératoire (110) et le récipient de collecte (130), dans lequel un trajet d'écoulement de liquide est défini à partir du champ opératoire (110), à travers la ligne de champ (120), et vers le récipient de collecte (130) ;
une ligne de vide (140) connectée entre la source de vide et le récipient de collecte (130), la source de vide étant conçue pour établir une aspiration pour aspirer du liquide du champ opératoire (110) le long du trajet d'écoulement du liquide ; et
- un ensemble filtre (160) disposé le long du trajet d'écoulement de liquide, l'ensemble filtre (160) comportant une membrane hydrophile conçue pour permettre le passage de liquide le long du trajet d'écoulement de liquide et empêcher le passage de l'air le long du trajet d'écoulement de liquide,
ou
- un ensemble soupape (1700) disposé le long du trajet d'écoulement de liquide, l'ensemble soupape comportant une chambre définissant une entrée conçue pour recevoir un liquide s'écoulant le long du trajet d'écoulement de liquide et une sortie conçue pour délivrer un fluide le long du trajet d'écoulement de liquide, l'ensemble soupape comportant une bille flottante disposée à l'intérieur de la chambre et conçue pour ouvrir et fermer sélectivement la sortie sur la base d'un niveau de liquide de la chambre, inhibant ainsi l'air de s'écouler hors de la sortie.

2. Système de collecte (100) selon la revendication 1, dans lequel, lorsque le système de collecte (100) comprend l'ensemble filtre (160), l'ensemble filtre (160) comporte en outre une membrane hydrophobe.

3. Système de collecte (100) selon la revendication 1 ou 2, dans lequel, lorsque le système de collecte (100) comprend l'ensemble filtre (160), l'ensemble filtre (160) comporte en outre un boîtier de corps de la membrane hydrophile à l'intérieur de celui-ci, le corps définissant une entrée et une sortie disposée le long du trajet d'écoulement de liquide.

4. Système de collecte (100) selon l'une quelconque revendication précédente, dans lequel, lorsque le système de collecte (100) comprend l'ensemble filtre (160), l'ensemble filtre (160) est disposé entre la ligne de champ (120) et le champ opératoire (110).

5. Système de collecte (100) selon l'une quelconque revendication précédente, dans lequel, lorsque le système de collecte (100) comprend l'ensemble filtre (160), l'ensemble filtre (160) est disposé entre la ligne de champ (120) et le récipient de collecte (130).

6. Système de collecte (100) selon l'une quelconque revendication précédente, dans lequel, lorsque le système de collecte (100) comprend l'ensemble filtre (160), l'ensemble filtre (160) est disposé à l'intérieur de la ligne de champ (120) vers une extrémité de ligne de champ de celui-ci ou vers une extrémité de récipient de collecte de celui-ci.

7. Système de collecte (100) selon l'une quelconque revendication précédente, comprenant en outre un réducteur de débit disposé le long du trajet d'écoulement de liquide en parallèle avec l'ensemble filtre (160), lorsque le système de collecte (100) comprend l'ensemble filtre (160).

8. Système de collecte (100) selon la revendication 1, dans lequel, lorsque le système de collecte (100) comprend l'ensemble soupape (1700), la ligne de champ (120) comporte une partie amont reliant le champ opératoire (110) à l'entrée de la chambre et une partie aval reliant la sortie de la chambre avec le récipient de collecte (130).
